# EUROPEAN PATENT APPLICATION

(11) **EP 4 556 005 A1**
(43) Date of publication of application: **21.05.2025**
(21) Application number: 23210758.1
(22) Date of filing: 17.11.2023
(51) Int. Cl.: A61K 31/165, A61K 31/315, A61K 31/343, A61K 31/52, A61K 45/06, A61P 7/00, A61P 25/00, A61P 31/14

(54) **COMPOUND FOR TREATING FLAVIVIRUS INFECTIONS**

(71) Applicant: Leibniz-Institut für Virologie, 20251 Hamburg (DE)
(72) Inventor: Scaturro, Pietro, 20251 Hamburg (DE); Rajasekharan, Sreejith, 20251 Hamburg (DE)
(74) Representative: Uexküll & Stolberg

(57) **Abstract**

The present invention relates to a compound which is effective in inhibiting the function of the ubiquitin-like modifier activating enzyme 5 (UBA5) for use in a method of treating or preventing a disease or condition mediated by a flavivirus infection. The invention is further directed to a pharmaceutical composition which is effective in inhibiting the function of UBA5 for use in a method for treating or preventing a disease or condition mediated by a flavivirus infection. The invention further relates to *in vitro* methods for identifying pharmaceutically active compounds that are useful for treating or preventing a disease or condition mediated by a flavivirus infection.

## Description

The present invention relates to a compound which is effective in inhibiting the function of the ubiquitin-like modifier activating enzyme 5 (UBA5) and the use of such compound in treating or preventing a disease or condition mediated by a flavivirus infection. The invention is further directed to a pharmaceutical composition which is effective in inhibiting the function of UBA5 for use in a method for treating or preventing a disease or condition mediated by a flavivirus infection. The invention further relates to *in vitro* methods for identifying pharmaceutically active compounds that are useful for treating or preventing a disease or condition mediated by a flavivirus infection.

### Background

Several of the most prevalent viral infections in the world are caused by flaviviruses, such as e.g. Dengue virus, Yellow fever virus, West Nile virus and Zika virus. Most of the flaviviruses are primarily transmitted to humans by arthropods which are typically endemic in tropical and subtropical regions of Africa, South America and Asia. Therefore, diseases caused by flaviviruses, such as vascular shock syndrome, encephalitis, microencephaly and fetal death, have so far mainly been a threat to the Global South. In recent years, however, as the earth continues to become warmer, the habitats of tropical and subtropical arthropods have started to spread, increasing also the risk from diseases mediated by flaviviruses in the Global North.

Thus, there is a great need for the treatment and prevention of diseases mediated by flaviviruses.

To date, the most widely used approach to control flavivirus infections is vaccination. Currently, there are licensed and commercially available vaccines for Yellow fever virus, Dengue virus, Japanese encephalitis virus, Kyasanur forest disease virus and Tick-borne encephalitis virus, and several vaccine candidates have been investigated in preclinical and clinical trials.

However, no antiviral compounds for treating diseases caused by flaviviruses have been approved until now. Recently, a drug candidate for the treatment of Dengue virus infections has entered clinical trials. The antiviral activity against Dengue viruses of this compound is based on preventing the interaction of the two Dengue virus proteins NS3 and NS4B, which results in the inhibition of Dengue virus replication (Kaptein et al., 2021, Nature, 598, 504-509). Since the active compound was developed specifically for targeting the NS3 and NS4B proteins of Dengue viruses, it is not effective against other flaviviruses.

Until now, it has not been known that the cellular protein "ubiquitin-like modifier activating enzyme 5" (UBA5) is a host dependency factor for flavivirus infections. UBA5 is involved in covalently conjugating the ubiquitin-like protein "ubiquitin fold modifier 1" (UFM1) to target proteins in an enzymatic reaction cascade that is referred to as "UFMylation". In this reaction cascade UBA5 has the role of the E1-type activating enzyme. It has been previously shown that UFMylation is associated with several cellular activities including the endoplasmatic reticulum stress response, hematopoiesis, fatty acid metabolism and the biogenesis of G-protein coupled receptors.

In two cases, UFMylation has been reported to affect infections of viruses which are, however, no flaviviruses. Firstly, UFMylation of the ribosomal protein RPL26 was shown to be required for efficient Hepatitis A virus replication (Kulsupatrakul et al., 2021, Cell Rep., 34(11), 108859). Moreover, the E3 ligase of the UFMylation cascade (UFL1) was reported to promote the antiviral immune response in Sendai virus infections (Snider et al., 2022, Proc. Natl. Acad. Sci. U.S.A., 119(15) :e2119531119). In these studies, however, no interaction between a viral protein and the UFM1-activating enzyme UBA5 was described. Instead, it was reported that the E3 ligase UFL1 of the UFMylation system is recruited to intracellular membranes following Sendai virus infection, suggesting a possible interaction between UFL1 and viral components.

UBA5-inhibitory compounds have been described in the art. The use of such compounds for treating or preventing a disease or condition mediated by a viral infection has, however, not been proposed.

The small molecule DKM2-93 (2-chloro-N-(3,4-dimethoxybenzyl)acetamide) has been reported to impair pancreatic cancer cell survival and *in vivo* tumor growth, thus making UBA5 a promising therapeutic target for treating pancreatic cancer (Roberts et al., 2017, ACS Chem. Biol., 12, 899-904).

WO 2015/179955 A1 describes UBA5 inhibitors with metal chelating moieties and the use of such inhibitors in disrupting cancer progression and in treating *Leishmania* parasite infections.

In addition, the natural product usenamine A was reported to exhibit UBA5-inhibitory effects and its use for preventing the progression of breast cancer was described (Fang et al., 2021, Biomolecules, 11, 1348).

The present invention is based on the objective of providing a compound for use in a method for the effective treatment or prevention of a wide range of flavivirus infections. It is desirable that, in contrast to a vaccine, the compound is effective in the treatment of a flavivirus infection even if the compound is administered only after viral infection has occurred. In addition, it is an object of the present invention to provide a compound that can prevent infections or the adverse consequences of infections when the compound is administered already prior to infection.

These objects are achieved by a compound for use in a method of treating or preventing a disease or condition mediated by a flavivirus infection according to claims 1 to 11. The invention also relates to the pharmaceutical compositions according to claims 12 and 13 and the *in vitro* methods according to claims 14 and 15.

### Inhibitory compounds

In a first aspect, the present invention relates to a compound which is effective in inhibiting the function of the ubiquitin-like modifier activating enzyme 5 (UBA5) for use in a method of treating or preventing a disease or condition mediated by a flavivirus infection in a subject.

The present invention is based on the surprising finding that flaviviral NS4B proteins interact with UBA5. It was, for example, found that UBA5 interacts with proteins from Zika virus, Dengue virus, Yellow fever virus, Powassan virus and Usutu virus. Importantly, it was found that the inhibition of UBA5 results in an inhibition of flavivirus replication. Hence, UBA5 was found to be a host dependency factor for flavivirus infections. In the light of the above findings, the present invention specifically provides the use of UBA5 inhibitors in a method of treating or preventing a disease or condition mediated by a flavivirus infection in a subject.

Most approved antiviral compounds, which are so far only available for treating viral infections of other viruses than flaviviruses, target viral proteins which are required for viral replication. But targeting host proteins that are involved in the virus replication cycle is a promising strategy for avoiding virus resistance and potentially leads to broad-spectrum anti-virals, since viruses from the same virus family often exploit the same cellular pathways.

The compounds according to the first aspect of the invention affect the function of a UBA5 protein, preferably the function of the human UBA5 protein. The terms "ubiquitin-like modifier activating enzyme 5" and "UBA5" refer to the E1-type activating enzyme that is encoded by the *UBA5* gene. Activation of UFM1 by UBA5 occurs in a two-step mechanism (Gerakis et al., Trends Cell Biol., 2019, Vol. 29, No. 12, 974-986). In a first reaction, UBA5 catalyzes the adenylation of the C-terminus of UFM1. This is followed by a second reaction in which UFM1 is attached to the cystein residue in the active site of UBA5. After activation, UFM1 is transferred from UBA5 to the E2 UEM1-conjugase UFC1 in a transthiolation reaction.

Two alternatively spliced transcript variants of UBA5 have been described. The nucleotide sequence of UBA5 is depicted in SEQ ID NO:1. The corresponding amino acid sequences of the transcript variants are depicted in SEQ ID NOs:2-3.

The compound according to the first aspect of the present invention is effective in inhibiting the function of UBA5. This means that the compound causes fewer products to be formed in one or more of the reactions, in which UBA5 is involved, than in the absence of the compound. For example, fewer products can be formed in one or more of (i) the adenylation of UFM1, (ii) the attachment of UFM1 to the active site of UBA5 and (iii) the transfer of UFM1 to UFC1. Alternatively or in addition, the compound may cause the amount of UBA5 to be reduced.

In a preferred embodiment, the compound of the first aspect of the invention decreases the UBA5 activity by at least 10%, at least 20%, at least 30%, at least 40%, at least 50%, at least 60%, at least 70%, at least 80%, at least 90% or at least 95% as compared to the activity in the absence of the compound. In a particularly preferred embodiment, the compound completely blocks the UBA5 activity such that there is no detectable transfer of UFM1 to UFC1.

Preferably, the compound according to the first aspect of the present invention is effective in inhibiting the function of a ubiquitin-like modifier enzyme (UBA5) comprising, or preferably consisting of, an amino acid sequence of any one of SEQ ID NOs:2-3, or an amino acid sequence having at least 80%, preferably at least 90%, sequence identity to at least one of the sequences of SEQ ID NOs:2-3.

UBA5 inhibition may be determined by any suitable assay. For example, UBA5 inhibition can be measured in an immunoblot assay (Zhou et al., STAR Protocols 3, 101074, March 18, 2022) or through an in vitro transthiolation assay (Sara et al., Bioorganic & Medicinal Chemistry Letters, Volume 26, Issue 18, 2016).

Preferably, the compound is specific for UBA5, i.e. the compound inhibits the function of UBA5, while it does essentially not inhibit the functions of other proteins or enzymes, such as other E1 activating enzymes. As a result of this UBA5 specificity, the compounds according to this embodiment elicit no or only tolerable side effects when administered to a subject.

The pharmaceutical composition according to the present invention is suitable for the treatment and prevention of flavivirus infections. The viral infections may be symptomatic or asymptomatic.

Treating a flavivirus infection may involve impairing the viral replication cycle, reducing the viral load in the infected subject and/or reducing the symptoms caused by the viral infection or by the infected person's immune response.

Preventing a flavivirus infection may involve reducing a subject's infectiousness, i.e. the likelihood for an infected subject to infect another individual, and/or a subject's susceptibility for infection, i.e. the likelihood for a subject to be infected by another infected individual.

According to the present invention, the compound may be intended for administration to a subject with an increased risk of being infected with a flavivirus, e.g. because the subject resides at an area with a high abundance of flavivirus vectors. The compound may also be intended for administration to a subject that is at a particular risk from the effects of the viral infection, e.g. due to pre-existing health conditions, high age or, particularly in the case of Zika virus infections, pregnancy.

The compound of the first aspect of the present invention may also be intended for treating a condition mediated by a flavivirus infection. Herein, term "condition" refers to a state which is not necessarily considered to be a disease, but which deviates from the healthy, uninfected state. For example, the condition mediated by a flavivirus infection may be an asymptomatic infection or a presymptomatic infection, i.e. an infection before the onset of symptoms.

The subject to which the compound of the invention is administered is preferably a mammal, and in particular a human. In a preferred embodiment, the subject is a pregnant woman. Particularly preferably, the subject is a pregnant woman and the compound is to be used in a method of treating or preventing a disease or condition mediated by a Zika virus infection.

The compound can be selected from all types of inorganic and organic molecules, including peptides, polypeptides, oligo- or polysaccharides, nucleic acids, fatty acids, steroids, small molecules and the like. For example, inhibitory compounds can be antisense DNA or RNA polynucleotides which bind to the *UBA5* gene and block transcription, RNA processing and/or translation of said gene. Also, the UBA5-inhibitory compound of the invention can be an RNA molecule which exerts its effect by RNA interference. Alternatively, the inhibitory compound of the invention can be a ribozyme that cleaves the UBA5-encoding mRNA. Other classes of molecules which may give rise to suitable UBA5-inhibitory compounds include peptides, antibodies and antibody fragments. Peptides that bind and interfere with the UBA5 protein may be conveniently screened in random peptide libraries.

It is preferred that the compound of the present invention is a small molecule. In particular, the compound has a molecular weight of 2,500 Da or less. Further preferred is a compound having a molecular weight of 2,000 Da or less, in particular 1,500 Da or less, more preferably 1,000 Da or less and particularly preferably 550 Da or less.

The UBA5-inhibitory compound is intended to be administered in an amount that is effective to inhibit the function of UBA5 in order to treat or prevent a disease or condition mediated by a flavivirus infection in a subject, e.g. by inhibiting flavivirus replication. This amount, which is also referred to as the "therapeutically effective amount" of the UBA5-inhibitory compound to be administered will depend on several parameters, such as the mode of administration, the particular type of flavivirus infection, the particular type of UBA5-inhibitory compound, the severity of the infection or disease or condition which is mediated by the flavivirus infection, and the age, height, weight, health, and physical condition of the subject to be treated. A therapeutically effective amount of the UBA5-inhibitory compound can be determined by one of ordinary skill in the art without undue experimentation given the disclosure set forth herein.

In a preferred embodiment, a therapeutically effective amount of a compound of the first aspect of the invention decreases flavivirus replication by at least 20%, in particular at least 50% and particularly preferably by at least 80%. For example, a therapeutically effective amount of a compound of the first aspect of the invention may decrease flavivirus replication by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, or at least 90%. The percentages of decrease of flavivirus replication relate in each case to the replication in absence in absence of the compound.

For treating of preventing a flavivirus infection, the UBA5-inhibitory compound will preferably be administered to the subject in an amount that ranges from 0.1 µg/kg body weight to 50,000 µg/kg body weight, e.g. from 0.5 µg/kg body weight to 30,000 µg/kg body weight, from 1.0 µg/kg body weight to 15,000 µg/kg body weight, from 5.0 µg/kg body weight to 10,000 µg/kg body weight, from 7.5 µg/kg body weight to 5,000 µg/kg body weight, from 10 µg/kg body weight to 2,500 µg/kg body weight, from 25 µg/kg body weight to 1,500 µg/kg body weight, from 50 µg/kg body weight to 1,000 µg/kg body weight, and more preferably from 100 µg/kg body weight to 800 µg/kg body weight.

In a preferred embodiment, the compound of the invention is used in a method of treating or preventing a disease or condition mediated by a flavivirus infection, wherein the flavivirus is selected from the group consisting of Zika virus, Japanese encephalitis virus, Powassan virus, West Nile virus, Yellow fever virus, Dengue virus, Usutu virus, and Tick-borne encephalitis virus, and preferably from the group consisting of Zika virus, Powassan virus, Yellow fever virus, Dengue virus, and Usutu virus.

In a particularly preferred embodiment, the flavivirus infection is a Zika virus infection.

The compound according to the first aspect of the invention is effective in inhibiting the function of UBA5. In a preferred embodiment, the compound is DKM2-93 (CAS No. 65836-72-8, 2-chloro-N-(3,4-dimethoxybenzyl)acetamide) .

In another preferred embodiment, the compound is usenamine A ((2*E*,9b*R*)-6-acetyl-2-(1-aminoethylidene)-7,9-dihydroxy-8,9*b*-di-methyldibenzofuran-1,3-dione) .

In even another preferred embodiment, the compound has the structure of Formula (I) 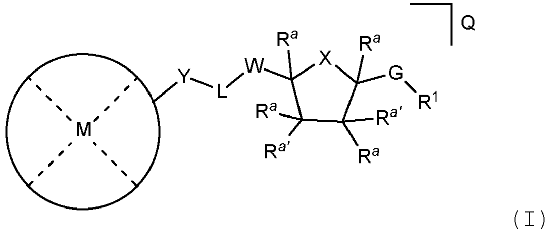 wherein 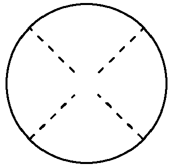 is a polyazamacrocycle chelating group,
- M: is a chelatable metal ion,
- Y: is -C(=O)- or -CH₂-,
- L: is
(i) (C₁-C₂₀)-alkylene, wherein
   (i.a) at least one of the carbon atoms is optionally replaced with a heteroatom selected from O, NR' and S, wherein R' is H, (C₁-C₆)-alkyl or -C(=O)-(C₁-C₆)-alkyl,
   (i.b) two or three adjacent carbon atoms are joined together to form a (C₃-C₁₀)-cycloalkyl group or -(C₆-C₁₀)-aryl group, and/or
   (i.c) the (C₁-C₂₀)-alkylene group is optionally substituted with at least one halo,
(ii) (C₂-C₂₀)-alkenylene, wherein
   (ii.a) at least one of the carbon atoms is optionally replaced with a heteroatom selected from O, NR' and S, wherein R' is H, (C₁-C₆)-alkyl or -C(=O)-(C₁-C₆)-alkyl,
   (ii.b) two or three adjacent carbon atoms are optionally joined together to form a (C₃-C₁₀)-cycloalkyl group or -(C₆-C₁₀)-aryl group, and/or
   (ii.c) the (C₂-C₂₀)-alkenylene group is optionally substituted with at least one halo,
      or
(iii) a polyethylene glycol (PEG) moiety,
- W: is
(i) -NH-C(=O)-,
(ii) -NR'-, wherein R' is H, (C₁-C₆)-alkyl or -C(=O)-(C₁-C₆)-alkyl,
- X: is
(i) -O-,
(ii) NR', wherein R' is H, (C₁-C₆)-alkyl or -C(=O)-(C₁-C₆)-alkyl,
(iii) -S-; or -S(=O)₂-, or
(iv) -C(R")₂, wherein each R" is independently or simultaneously H, halo or (C₁-C₆)-alkyl,
- R*^{a}* and R*^{a'}*: are each independently or simultaneously
(i) H,
(ii) OH,
(iii) halo, or
(iv) (C₁-C₃)-alkyl, and
- G is: (i) O,
(ii) S,
(iii) NR²,
- R¹ and R²: are each independently or simultaneously
(i) H,
(ii) (C₁-C₆)-alkyl,
(iii) (C₃-C₁₀)-cycloalkyl,
(iv) (C₃-C₁₀)-heterocycloalkyl,
(v) -(CH₂)ₙ-(C₆-C₁₀)-aryl,
(vi) -(CH₂)ₙ-(C₅-C₁₀)-heteroaryl,
or
- R¹ and R²: are joined together to form a
(vii) guanine or a guanine derivative,
(viii) cytosine or a cytosine derivative,
(ix) thymine or a thymine derivative,
(x) adenine or an adenine derivative,
- and Q: is any suitable counteranion,
or a solvate, prodrug and/or stereoisomer thereof.

As used herein, the term "polyazamacrocycle chelating group" refers to a macrocycle containing at least 8 atoms, at least two of which (preferably 3, 4, 5 or 6 atoms) are nitrogen atoms, and which is able to chelate a metal ion. The polyazamacrocycle chelating group may also have carboxylic acid substituents, such as ethanoic substituents, bound to the nitrogen atoms. Polyazamacrocycle chelating groups as well as suitable chelatable metal ions, which are preferred according to the present invention, are described in WO 2015/179955 A1, particularly in paragraphs [0048], [0049] and [0060]-[0064] and in the claims.

Further groups M, Y, L, W, G, X, Rₐ, R_{a'}, R₁, which are preferred according to the present invention, are also described in WO 2015/179955 A1, particularly in the claims.

It is further preferred that the compound has the structure of 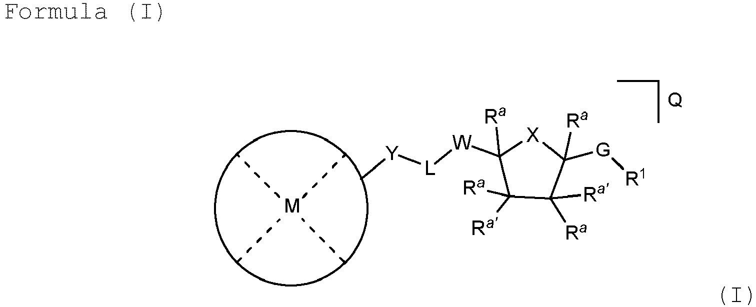 wherein 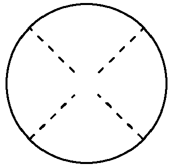 is a polyazamacrocycle chelating group with the structure 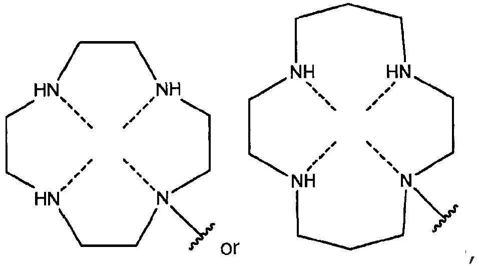
- M: is Zn²⁺, Cu²⁺, Co²⁺, Pd²⁺, Gd²⁺, Tb²⁺, Eu⁺ or Mo²⁺, preferably Zn²⁺ or Cu²⁺,
- Y: is -C(=O)- or -CH₂-,
- L: is
(i) (C₁-C₆)-alkylene, preferably (C₃-C₆)-alkylene, wherein
   (i.a) one to three of the carbon atoms are optionally replaced with a heteroatom selected from O, NR' and S, wherein R' is H, (C₁-C₆)-alkyl or -C(=O)-(C₁-C₆)-alkyl,
   (i.b) two or three adjacent carbon atoms are joined together to form a cyclohexyl group or phenyl group, and/or
   (i.c) the alkylene group is optionally substituted with at least one fluoro,
      or
(ii) a polyethylene glycol (PEG) moiety,
- W: is
(i) -NH-C(=O)-,
(ii) -NR'-, wherein R' is H, (C₁-C₆)-alkyl or -C(=O)-(C₁-C₆)-alkyl, 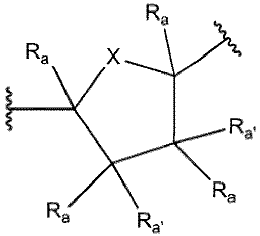
has one of the following structures 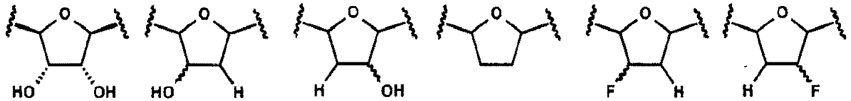 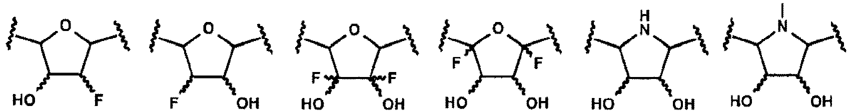 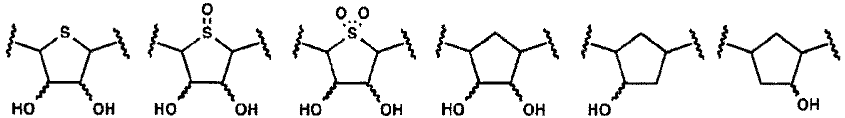 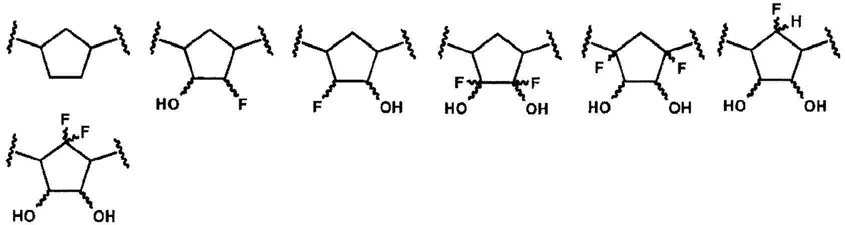
- G is: (i) O,
(ii) S,
(iii) NR², preferably NR²,
- R¹ and R²: are each independently or simultaneously
(i) H,
(ii) (C₅-C₇)-cycloalkyl, preferably cyclopentyl or cyclohexyl,
(iii) (C₅-C₇)-heterocycloalkyl, Preferably morpholinyl or piperazinyl,
(iv) phenyl or -(CH₂)-phenyl,
(v) naphthyl or -(CH₂)-naphthyl,
(vi) -(CH₂)ₙ-(C₅-C₆)-heteroaryl,
or
- R¹ and R²: are joined together to form a
(vii) guanine or a guanine derivative,
(viii) cytosine or a cytosine derivative,
(ix) thymine or a thymine derivative,
(x) adenine or an adenine derivative,
and wherein R¹ and R² are particularly preferably joined together to form an adenosine derivative having the structure 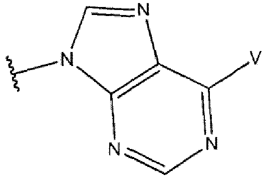 wherein V is H, halo, (C₁-C₆)-alkyl, (C₆-C₁₀)-aryl, (C₅-C₁₀)-heteroaryl, (C₃-C₁₀)-cycloalkyl, (C₃-C₁₀)-heterocycloalkyl,
and Q is any suitable counteranion,
or a solvate, prodrug and/or stereoisomer thereof.

In a particularly preferred embodiment, the compound has the structure of formula (II) 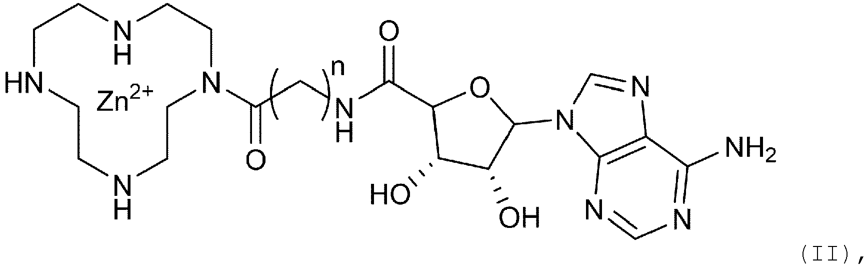 wherein n is an integer in the range of 1 to 5, and preferably is 4 or 5, particularly preferably 5.

In another preferred embodiment, the compound is a stereoisomer of the structure of formula (II), wherein n is in particular 4 or 5, more preferably 5.

Hence, the UBA5-inhibitory compound may be selected from the group consisting of DKM2-93 (CAS No. 65836-72-8), usenamine A, and the compound with the structure of formula (I), 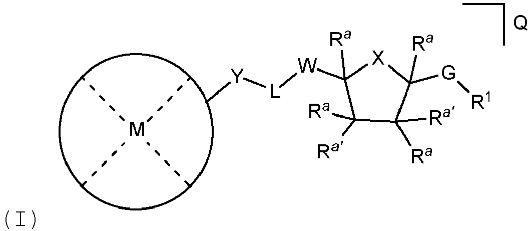 wherein M, Y, L, W, G, X, Rₐ, R_{a'}, R₁ are as defined above.

### Pharmaceutical composition

The compound of the present invention that inhibits UBA5 is normally provided in the form of a pharmaceutical composition. Therefore, in a second aspect, the invention relates to a pharmaceutical composition comprising the compound of the first aspect of the invention, or its pharmaceutically acceptable salt, solvate, prodrug or ester, for use in a method for treating or preventing a disease or condition mediated by a flavivirus infection in a subject. The pharmaceutical composition of the present invention comprises an amount of the UBA5-inhibitory compound that is effective to inhibit the function of UBA5 in order to inhibit flavivirus replication.

The pharmaceutical composition of the invention normally comprises one or more excipients. Preferably the pharmaceutical composition comprises one or more excipients selected from the group consisting of diluents, solvents, binders, carriers, lubricants, fillers, glidant, coatings, surfactants, stabilizing agents, antitacking agents, preservatives, emulsifiers, dispersants, wetting agents, and adjuvants. Generally, the pharmaceutical composition may be administered in any suitable form that does not interfere with the compound's inhibitory activity.

Suitable pharmaceutical formulations can be determined by the skilled person depending on the route of administration and the desired dosage. See, e.g., Remington's Pharmaceutical Sciences, 1435-712 (18th ed., Mack Publishing Co, Easton, Pennsylvania, 1990). Formulations may influence the physical state, stability, rate of *in vivo* release and rate of *in vivo* clearance of the administered agents. Depending on the route of administration, a suitable dose may be calculated according to body weight, body surface areas or organ size. Further refinement of the calculations necessary to determine the appropriate treatment dose is routinely made by those of ordinary skill in the art without undue experimentation, especially in light of the dosage information and assays disclosed herein as well as the pharmacokinetic data obtainable through animal or human clinical trials.

The compound can be present in a pharmaceutical composition as a pharmaceutically acceptable salt. As used herein, "pharmaceutically acceptable salts" include, for example, base addition salts and acid addition salts.

Pharmaceutically acceptable base addition salts may be formed with metals or amines, such as alkali and alkaline earth metals or organic amines. Pharmaceutically acceptable salts of compounds may also be prepared with a pharmaceutically acceptable cation. Suitable pharmaceutically acceptable cations are well known to those skilled in the art and include alkaline, alkaline earth, ammonium and quaternary ammonium cations. Carbonates or hydrogen carbonates are also possible. Examples of metals used as cations are sodium, potassium, magnesium, ammonium, calcium, or ferric, and the like. Examples of suitable amines include isopropylamine, trimethylamine, histidine, N,N'-dibenzylethylenediamine, chloroprocaine, choline, diethanolamine, dicyclohexylamine, ethylenediamine, N-methylglucamine, and procaine.

Pharmaceutically acceptable acid addition salts include inorganic or organic acid salts. Examples of suitable acid salts include the hydrochlorides, formates, acetates, citrates, salicylates, nitrates, phosphates. Other suitable pharmaceutically acceptable salts are well known to those skilled in the art and include, for example, formic, acetic, citric, oxalic, tartaric, or mandelic acids, hydrochloric acid, hydrobromic acid, sulfuric acid or phosphoric acid; with organic carboxylic, sulfonic, sulfo or phospho acids or N-substituted sulfamic acids, for example acetic acid, trifluoroacetic acid (TFA), propionic acid, glycolic acid, succinic acid, maleic acid, hydroxymaleic acid, methylmaleic acid, fumaric acid, malic acid, tartaric acid, lactic acid, oxalic acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid or isonicotinic acid; and with amino acids, such as the 20 alpha amino acids involved in the synthesis of proteins in nature, for example glutamic acid or aspartic acid, and also with phenylacetic acid, methanesulfonic acid, ethanesulfonic acid, 2 -hydroxy ethanesulfonic acid, ethane 1,2-disulfonic acid, benzenesulfonic acid, 4-methylbenzenesulfonic acid, naphthalene 2-sulfonicacid, naphthalene 1,5-disulfonic acid, 2- or 3-phosphogly cerate, glucose 6-phosphate, N-cyclohexylsulfamic acid (with the formation of cyclamates), or with other acid organic compounds, such as ascorbic acid.

Pharmaceutical compositions containing the compounds disclosed herein can be manufactured in a conventional manner, e.g., by conventional mixing, dissolving, granulating, dragee-making, levigating, emulsifying, encapsulating, entrapping, or lyophilizing processes. Proper formulation is dependent upon the route of administration chosen.

The pharmaceutical composition of the present invention may be formulated for oral administration.

For oral administration, suitable compositions can be formulated readily by combining the compound of the first aspect of the invention with pharmaceutically acceptable excipients such as carriers well known in the art. Such excipients and carriers enable the present compounds to be formulated as tablets, pills, dragees, capsules, liquids, gels, syrups, slurries, suspensions and the like, for oral ingestion by a patient to be treated. Pharmaceutical preparations for oral use can be obtained by adding the compound of the first aspect of the invention with a solid excipient, optionally grinding a resulting mixture, and processing the mixture of granules, after adding suitable auxiliaries, if desired, to obtain tablets or dragee cores. Suitable excipients include, for example, fillers and cellulose preparations. If desired, disintegrating agents can be added. Pharmaceutically acceptable ingredients are well known for the various types of formulation and may be for example binders (e.g., natural or synthetic polymers), lubricants, surfactants, sweetening and flavoring agents, coating materials, preservatives, dyes, thickeners, adjuvants, antimicrobial agents, antioxidants and carriers for the various formulation types.

When a therapeutically effective amount of the compound of the first aspect is administered orally, the composition typically is in the form of a solid (e.g., tablet, capsule, pill, powder, or troche) or a liquid formulation (e.g., aqueous suspension, solution, elixir, or syrup).

When administered in tablet form, the composition can additionally contain a functional solid and/or solid carrier, such as a gelatin or an adjuvant. The tablet, capsule, and powder can contain about 1 to about 95% by weight of the compound of the first aspect, and preferably from about 15 to about 90% by weight of the compound of the first aspect.

When administered in liquid or suspension form, a functional liquid and/or a liquid carrier such as water, petroleum, or oils of animal or plant origin can be added. The liquid form of the composition can further contain physiological saline solution, sugar alcohol solutions, dextrose or other saccharide solutions, or glycols. When administered in liquid or suspension form, the composition can contain about 0.5 to about 90% by weight of the compound of the first aspect, and preferably about 1 to about 50% of the compound of the first aspect. In one embodiment contemplated, the liquid carrier is non-aqueous or substantially non-aqueous. For administration in liquid form, the composition may be supplied as a rapidly-dissolving solid formulation for dissolution or suspension immediately prior to administration.

In another embodiment, the pharmaceutical composition of the present invention may be formulated for parenteral administration.

When a therapeutically effective amount of the compound of the first aspect of the invention is administered by intravenous, cutaneous, or subcutaneous injection, the composition is in the form of a pyrogen-free, parenterally acceptable aqueous solution. The preparation of such parenterally acceptable solutions, having due regard to pH, isotonicity, stability, and the like, is within the skill in the art. A preferred composition for intravenous, cutaneous, or subcutaneous injection typically contains, in addition to a compound disclosed herein, an isotonic vehicle. Such compositions may be prepared for administration as solutions of free base or pharmacologically acceptable salts in water suitably mixed with a surfactant, such as hydroxypropylcellulose. Dispersions also can be prepared in glycerol, liquid polyethylene glycols, and mixtures thereof and in oils. Under ordinary conditions of storage and use, these preparations can optionally contain a preservative to prevent the growth of microorganisms.

The compounds of first aspect can be formulated for parenteral administration by injection (e.g., by bolus injection or continuous infusion). Formulations for injection can be presented in unit dosage form (e.g., in ampules or in multidose containers), with an added preservative. The compositions can take such forms as suspensions, solutions, or emulsions in oily or aqueous vehicles, and can contain formulatory agents such as suspending, stabilizing, and/or dispersing agents.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the embodiment of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Injectable compositions can include sterile aqueous solutions, suspensions, or dispersions and sterile powders for the extemporaneous preparation of sterile injectable solutions, suspensions, or dispersions. In all embodiments the form must be sterile and must be fluid to the extent that easy syringability exists. It must be stable under the conditions of manufacture and storage and must resist the contaminating action of microorganisms, such as bacteria and fungi, by optional inclusion of a preservative. The carrier can be a solvent or dispersion medium containing, for example, water, ethanol, polyol (e.g., glycerol, propylene glycol, and liquid polyethylene glycol, and the like), suitable mixtures thereof, and vegetable oils. In one embodiment contemplated, the carrier is non-aqueous or substantially non-aqueous. The proper fluidity can be maintained, for example, by the use of a coating, such as lecithin, by the maintenance of the required particle size of the compound in the embodiment of dispersion and by the use of surfactants. The prevention of the action of microorganisms can be brought about by various antibacterial and antifungal agents, for example, parabens, chlorobutanol, phenol, sorbic acid, thimerosal, and the like. In many embodiments, it will be preferable to include isotonic agents, for example, sugars or sodium chloride. Prolonged absorption of the injectable compositions can be brought about by the use in the compositions of agents delaying absorption, for example, aluminum monostearate and gelatin.

Pharmaceutical formulations for parenteral administration include aqueous solutions of the compounds in water-soluble form. Additionally, suspensions of the compounds can be prepared as appropriate oily injection suspensions. Suitable lipophilic solvents or vehicles include fatty oils or synthetic fatty acid esters. Aqueous injection suspensions can contain substances which increase the viscosity of the suspension. Optionally, the suspension also can contain suitable stabilizers or agents that increase the solubility of the compounds and allow for the preparation of highly concentrated solutions. Alternatively, a present composition can be in powder form for constitution with a suitable vehicle (e.g., sterile pyrogen-free water) before use.

For administration by inhalation, compounds of the first aspect of the present invention are conveniently delivered in the form of an aerosol spray presentation from pressurized packs or a nebulizer, with the use of a suitable propellant. In the embodiment of a pressurized aerosol, the dosage unit can be determined by providing a valve to deliver a metered amount. Capsules and cartridges of, e.g., gelatin, for use in an inhaler or insufflator can be formulated containing a powder mix of the compound and a suitable powder base such as lactose or starch.

Compounds of the first aspect can also be formulated in rectal compositions, such as suppositories or retention enemas (e.g., containing conventional suppository bases). In addition to the formulations described previously, the compounds also can be formulated as a depot preparation. Such long-acting formulations can be administered by implantation (e.g., subcutaneously or intramuscularly) or by intramuscular injection. Thus, for example, the compounds can be formulated with suitable polymeric or hydrophobic materials (for example, as an emulsion in an acceptable oil) or ion exchange resins, or as sparingly soluble derivatives, for example, as a sparingly soluble salt.

In particular, a compound of the first aspect of the invention can be administered orally, buccally, or sublingually in the form of tablets containing excipients, such as starch or lactose, or in capsules or ovules, either alone or in admixture with excipients, or in the form of elixirs or suspensions containing flavoring or coloring agents. Such liquid preparations can be prepared with pharmaceutically acceptable additives, such as suspending agents. A compound also can be injected parenterally, for example, intravenously, intramuscularly, subcutaneously, or intracoronarily. For parenteral administration, the compound is best used in the form of a sterile aqueous solution which can contain other substances, for example, salts, or sugar alcohols, such as mannitol, or glucose, to make the solution isotonic with blood.

Slow release or sustained release formulations may also be prepared in order to achieve a controlled release of the active compound in contact with the body fluids in the gastrointestinal (GI) tract, and to provide a substantially constant and effective level of the active compound in the blood plasma. For example, release can be controlled by one or more of dissolution, diffusion, and ion-exchange. In addition, the slow release approach may enhance absorption via saturable or limiting pathways within the GI tract. For example, the compound may be embedded for this purpose in a polymer matrix of a biological degradable polymer, a water-soluble polymer or a mixture of both, and optionally suitable surfactants. Embedding can mean in this context the incorporation of micro-particles in a matrix of polymers. Controlled release formulations are also obtained through encapsulation of dispersed micro-particles or emulsified micro-droplets via known dispersion or emulsion coating technologies.

The amount of compound administered can be dependent on the subject being treated, on the subject's age, health, sex, and weight, the kind of concurrent treatment (if any), severity of the affliction, the nature of the effect desired, the manner and frequency of treatment, and the judgment of the prescribing physician. The frequency of dosing also can be dependent on pharmacodynamic effects on arterial oxygen pressures. However, the most preferred dosage can be tailored to the individual subject, as is understood and determinable by one of skill in the art, without undue experimentation. This typically involves adjustment of a standard dose (e.g., reduction of the dose if the patient has a low body weight).

While individual needs vary, determination of optimal ranges of effective amounts of the compound is within the skill of the art. For administration to a human in the curative or prophylactic treatment of the conditions and disorders identified herein, for example, typical dosages of the compounds of the present invention can be about 0.01 mg/kg/day to about 50 mg/kg/day, for example at least 0.05 mg/kg, at least 0.08 mg/kg, at least 0.1 mg/kg, at least 0.2 mg/kg, at least 0.3 mg/kg, at least 0.4 mg/kg, or at least 0.5 mg/kg, and preferably 50 mg/kg or less, 40 mg/kg or less, 30 mg/kg or less, 20 mg/kg or less, or 10 mg/kg or less, which can be about 2.5 mg/day (0.5 mg/kg × 5kg) to about 5000 mg/day (50mg/kg × 100kg), for example. For example, dosages of the compounds can be about 0.1 mg/kg/day to about 50 mg/kg/day, about 0.05 mg/kg/day to about 10 mg/kg/day, about 0.05 mg/kg/day to about 5 mg/kg/day, about 0.05 mg/kg/day to about 3 mg/kg/day, about 0.07 mg/kg/day to about 3 mg/kg/day, about 0.09 mg/kg/day to about 3 mg/kg/day, about 0.05 mg/kg/day to about 0.1 mg/kg/day, about 0.1 mg/kg/day to about 1 mg/kg/day, about 1 mg/kg/day to about 10 mg/kg/day, about 1 mg/kg/day to about 5 mg/kg/day, about 1 mg/kg/day to about 3 mg/kg/day, about 3 mg/day to about 500 mg/day, about 5 mg/day to about 250 mg/day, about 10 mg/day to about 100 mg/day, about 3 mg/day to about 10 mg/day, or about 100 mg/day to about 250 mg/day. Such doses may be administered in a single dose or it may be divided into multiple doses.

### In vitro screening method

The finding that UBA5-inhibitory compounds inhibit flavivirus replication in a subject allows the design of screening assays which identify pharmaceutically active compounds which are effective in treating and/or preventing a disease or condition mediated by a flavivirus infection in a subject. Thus, the present invention also provides methods for identifying a pharmaceutically active compound that could be used for treating or preventing a disease or condition mediated by a flavivirus infection in a subject. The *in vitro* methods of the invention can be used for drug screening approaches that aim to identify new pharmaceutically active compounds for treating or preventing a disease or condition mediated by a flavivirus infection.

In a third aspect, the invention relates to an *in vitro* method for identifying a pharmaceutically active compound for treating or preventing a disease or condition mediated by a flavivirus infection in a subject, comprising
(a) contacting a candidate compound with a ubiquitin-like modifier activating enzyme 5 (UBA5),
(b) detecting whether the candidate compound inhibits the function of UBA5,
wherein a compound which inhibits the function of UBA5 is suitable for treating or preventing a disease or condition mediated by a flavivirus infection in a subject.

Contacting a candidate compound with UBA5 may, for example, take place in a cell culture system.

UBA5 inhibition may be measured in an immunoblot assay (Zhou et al., STAR Protocols 3, 101074, March 18, 2022). The UBA5 preferably comprises, in particular consists of, an amino acid sequence of any one of SEQ ID Nos:2-3, or an amino acid sequence having at least 80%, in particular at least 90%, sequence identity to at least one of the sequences of SEQ ID Nos:2-3.

In an alternative approach, the screening can target compounds which interfere with flavivirus replication. The invention therefore relates, in a fourth aspect, to an *in vitro* method for identifying a pharmaceutically active compound for treating or preventing a disease or condition mediated by a flavivirus infection in a subject, comprising
(a) contacting a candidate compound with a cell that expresses a functional ubiquitin-like modifier activating enzyme 5 (UBA5),
(b) infecting the cell with a flavivirus before or after the cell was contacted with the candidate compound,
(c) detecting whether the candidate compound decreases the replication of the flavivirus in the cell, compared to a cell which was infected with the flavivirus but not contacted with the candidate compound,
wherein a compound which decreases the replication of a flavivirus in the cell is suitable for treating or preventing a disease or condition mediated by a flavivirus infection in a subject.

Whether the proteins expressed in the cell are functional or not can be determined in an immunoblot assay (Zhou et al., STAR Protocols 3, 101074, March 18, 2022). Proteins are considered functional if they at least one of the known reaction products of UBA5 can be detected.

Typically, contacting a candidate compound with a cell that expresses functional UBA5 in step (a) also includes that the candidate compound is contacted with UBA5.

The inhibition of flavivirus replication may be determined by any suitable assay. For example, flavivirus replication may be determined using a Renilla luciferase reporter assay. The UBA5 preferably comprises, in particular consists of, an amino acid sequence of any one of SEQ ID Nos:2-3, or an amino acid sequence having at least 80%, in particular at least 90%, sequence identity to at least one of the sequences of SEQ ID Nos:2-3.

Thus, the *in vitro* methods of the third and fourth aspect of the invention include methods, in which the candidate compound is a compound of the first aspect of the invention that is effective in inhibiting the function of UBA5.

The invention disclosed herein is exemplified by the following examples which should not be construed to limit the scope of the invention. Alternative mechanistic pathways and analogous structures will be apparent to those skilled in the art.

### Examples

### a) UBA5 interacts with the NS4B protein of multiple flaviviruses

The interaction of UBA5 with NS4B proteins of flaviviruses was studied by label-free quantitative proteomics using the iBAQ (intensity-based absolute quantification) method.

For sample preparation, JEG-3 human placental cells stably expressing HA-tagged 2k-NS4B proteins of Zika virus (ZIKV, strain H/PF/2013), Dengue virus (DENV-2, strain 16681), Powassan virus (POWV, strain LB), Usutu virus (USUV, strain Vienna 2001), Yellow fever virus (YFV, strain Asibi) or control proteins (Gaussia luciferase, Gluc; NS4B of Hepatitis C virus (HCV, Jfh-1 strain) were lysed and clarified supernatants were used for HA-specific affinity purification using anti-HA-conjugated agarose beads. After extensive washing, proteins bound to the resin were denatured by incubation in 40 µl Urea/Thiourea buffer (6 M Urea/2 M thiourea in 10 mM HEPES, pH 8.0), and reduced and alkylated in 10 mM DTT and 55 mM iodoacetamide, respectively. NS4B- or control-interacting proteins were proteolytically digested with 1 µg LysC (FUJIFILM Wako Chemicals) and 1 µg trypsin (Promega) in 40mM ABC buffer (50mM NH4HCO3 in water, pH 8.0) overnight at 25°C. After digestion, peptides were purified on stage tips with 3 layers of C18 Empore filter discs (3M) as previously described (PubMed identifier (PMID): 30177828).

Samples were analysed on a nanoElute (plug-in v.1.1.0.27; Bruker) coupled to a trapped ion mobility spectrometry quadrupole time of flight (timsTOF Pro) (Bruker) equipped with a CaptiveSpray source. Peptides were injected into a Trap cartridge (5mm×300µm, 5µm C18; Thermo Fisher Scientific) and next separated on a 25cmx75pm analytical column, 1.6µm C18 beads with a packed emitter tip (IonOpticks). The column temperature was maintained at 50°C using an integrated column oven (Sonation GmbH). The column was equilibrated using 4 column volumes before loading samples in 100% buffer A (99.9% Milli-Q water, 0.1% formic acid (FA)). Samples were separated at 400nl min⁻¹ using a linear gradient from 2 to 17% buffer B (99.9% ACN, 0.1% FA) over 60min before ramping up to 25% (30min), 37% (10min) and 95% of buffer B (10min) and sustained for 10min (total separation method time, 120min). The timsTOF Pro was operated in parallel accumulation-serial fragmentation (PASEF) mode using Compass Hystar v.5.0.36.0. Settings were as follows: mass range 100-1700m/z, 1/K0 Start 0.6V·s/cm² End 1.6V·s/cm²; ramp time 110.1ms; lock duty cycle to 100%; capillary voltage 1,600V; dry gas 31min⁻¹; dry temperature 180°C. The PASEF settings were: 10 tandem mass spectrometry (MS) scans (total cycle time, 1.27s); charge range 0-5; active exclusion for 0.4min; scheduling target intensity 10,000; intensity threshold 2,500; collision-induced dissociation energy 42eV.

Raw MS data were processed with the MaxQuant software v.1.6.17.0 using the built-in label-free quantitation algorithm and Andromeda search engine (PMID: 27809316). The search was done against the Homo sapiens proteome containing forward and reverse sequences plus the exogenous HA-tagged baits. Additionally, the intensity-based absolute quantification (iBAQ) algorithm and match between runs option were used. In MaxQuant, carbamidomethylation was set as fixed and methionine oxidation and N-acetylation as variable modifications. Initial search peptide tolerance was set at 20 p.p.m. and the main search was set at 10 p.p.m.. Experiment type was set as TIMS-DDA with no modification to the default settings. Search results were filtered with a false discovery rate of 0.01 for peptide and protein identification.

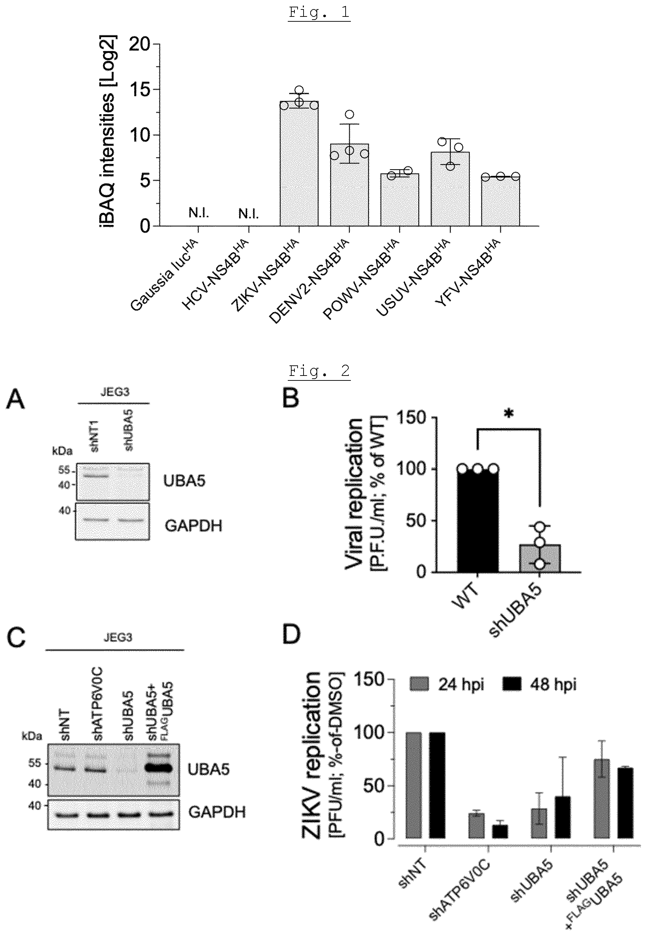

Results were plotted as heat map using Perseus (PMID: 27348712) and are shown in Figure 1, which shows the relative intensity of human UBA5 identified across the viral or control baits. Each column represents an individual biological replicate. (N.I.=Not identified; n=3-4)

Thus, it was found that UBA5 interacts with NS4B proteins from multiple flaviviruses (Zika virus, Dengue virus, Powassan virus, Usutu virus, Yellow fever virus).

### b) Zika virus replication is inhibited upon silencing of UBA5

UBA5 was found to be a Zika virus host-dependency factor. Silencing UBA5 was shown to inhibit Zika virus replication.

JEG-3 human placental cells stably expressing control non-targeting (NT) shRNA (short-hairpins RNA) or shRNAs against UBA5 were analyzed for efficiency of protein expression knock-down by western-blotting using anti-UBA5 or anti-GADPH-specific antibodies. The results are shown in Figure 2A and confirm strongly reduced production of UBA5, i.e. effective silencing of UBA5.

The effect of silencing of UBA5 on Zika virus replication was studied (see Figure 2B). Wild-type or stable knock-down JEG-3 cells depleted of UBA5 were seeded in 24-well plates (8×10⁴ cells/well) . The day after cell monolayers were infected with ZIKV (PRVABC59 strain) at an MOI (Multiplicity of Infection) of 0.01 and 48h later virus-containing supernatants were analyzed by plaque-forming unit assay (PFU) on VeroE6 cells. (n=4; Mann-Whitney test p-value < 0.05). Thus, silencing of UBA5 resulted in a strong reduction of Zika virus replication.

Specificity of inhibition of Zika virus replication as a result of silencing UBA5 was further confirmed in a "rescue" experiment, in which stable shUBA5 JEG-3 cells from were reconstituted with shRNA-resistant exogenous Flag-tagged UBA5 and infected with ZIKV (PRVABC59 strain) as described above. Stable JEG-3 cells silenced with non-targeting shRNAs (NT) or ATP6V0C- and UBA5-specific shRNAs were used as negative and positive controls, respectively. Reconstitution with Flag-tagged UBA5 was found to result in UBA5 protein expression (see Figure 2C) and high Zika virus replication (see Figure 2D).

### c) UBA5 inhibitor DKM29-3 effectively inhibits Zika virus replication

The effects of a pharmacological modulation of UFMylation, in particular by UBA5 inhibition, on Zika virus replication were studied.

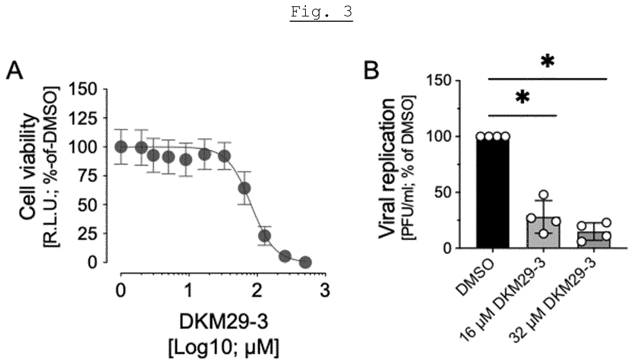

In a first experiment, the cytotoxicity of the UBA5 inhibitor DKM29-3 was determined. Naive JEG-3 human placental cells were seeded in 24-well plates (8×10⁴ cells/well) and on the next day treated with increasing amounts of the UBA5 inhibitor DKM29-3. Forty-height hours later, cell viability was assessed by Resazurin assay using a multi-well plate reader to determine the 50% cytotoxicity concentration (CC50=80.23 µM) (see Figure 3A).

Then, the effect of UBA5-inhibitor DKM29-3 on Zika virus replication was studied. Wild-type JEG-3 cells were seeded as described above and infected with ZIKV (PRVABC59 strain) at an MOI (Multiplicity of Infection) of 0.01 for 1h at 37°C. After virus absorption the cells monolayers were washed with PBS (Phosphate Buffer Saline), and cell culture media containing 16 µM or 32 µM of DKM29-3 were added to the cells. Forty-height hours later virus-containing supernatants were analyzed by plaque-forming unit assay (PFU) on VeroE6 cells (n=4; Mann-Whitney test p-value < 0.05). Zika virus replication in cells treated with DKM29-3 was found to be significantly reduced in presence of DKM29-3.

## Claims

1. Compound which is effective in inhibiting the function of ubiquitin-like modifier activating enzyme 5 (UBA5) for use in a method of treating or preventing a disease or condition mediated by a flavivirus infection in a subject.

2. Compound for use according to claim 1, wherein the subject is a mammal, preferably a human.

3. Compound for use according to claim 1 or 2, wherein the compound is a small molecule.

4. Compound for use according to any one of claims 1 to 3, wherein the compound has a molecular weight of 2,500 Da or less.

5. Compound for use according to any one of claims 1 to 4, wherein the compound is to be administered in an amount of from 0.1 µg/kg body weight to 50,000 µg/kg body weight.

6. Compound for use according to any one of claims 1 to 5, wherein the flavivirus infection is an infection caused by a flavivirus selected from the group consisting of Zika virus, Japanese encephalitis virus, Powassan virus, West Nile virus, Yellow fever virus, Dengue virus, Usutu virus and Tick-borne encephalitis virus.

7. Compound for use according to claim 6, wherein the flavivirus infection is a Zika virus infection.

8. Compound for use according to any one of claims 1 to 7, wherein the compound is selected from the group consisting of DKM2-93 (CAS No. 65836-72-8, 2-chloro-N-(3,4-dimethoxybenzyl)acetamide), usenamine A ((2E,9bR)-6-acetyl-2-(1-aminoethylidene)-7,9-dihydroxy-8,9b-dimethyldibenzofuran-1,3-dione), and compounds with the structure of Formula (I) 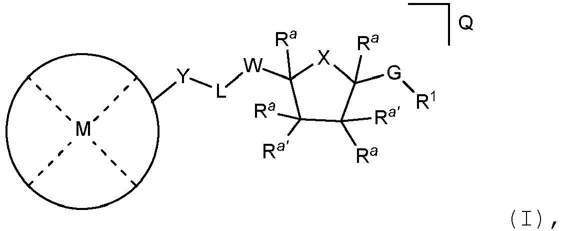 wherein 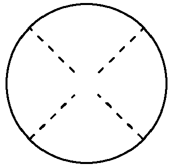 is a polyazamacrocycle chelating group,
M is a chelatable metal ion,
Y is -C(=O)- or -CH₂-,
L is
(i) (C₁-C₂₀)-alkylene, wherein
(i.a) at least one of the carbon atoms is optionally replaced with a heteroatom selected from O, NR' and S, wherein R' is H, (C₁-C₆)-alkyl or-C(=O)-(C₁-C₆)-alkyl,
(i.b) two or three adjacent carbon atoms are joined together to form a (C₃-C₁₀)-cycloalkyl group or -(C₆-C₁₀)-aryl group, and/or
(i.c) the (C₁-C₂₀)-alkylene group is optionally substituted with at least one halo,
(ii) (C₂-C₂₀)-alkenylene, wherein
(ii.a) at least one of the carbon atoms is optionally replaced with a heteroatom selected from O, NR' and S, wherein R' is H, (C₁-C₆)-alkyl or -C(=O)-(C₁-C₆)-alkyl,
(ii.b) two or three adjacent carbon atoms are optionally joined together to form a (C₃-C₁₀)-cycloalkyl group or -(C₆-C₁₀)-aryl group, and/or
(ii.c) the (C₂-C₂₀)-alkenylene group is optionally substituted with at least one halo,
or
(iii) a polyethylene glycol (PEG) moiety,
W is
(i) -NH-C(=O)-,
(ii) -NR'-, wherein R' is H, (C₁-C₆)-alkyl or -C(=O)-(C₁-C₆)-alkyl,
X is
(i) -O-,
(ii) NR', wherein R' is H, (C₁-C₆)-alkyl or -C(=O)-(C₁-C₆)-alkyl,
(iii) -S-; or -S(=O)₂-, or
(iv) -C(R")₂, wherein each R" is independently or simultaneously H, halo or (C₁-C₆)-alkyl,
R*^{a}* and R*^{a'}* are each independently or simultaneously
(i) H,
(ii) OH,
(iii) halo, or
(iv) (C₁-C₃)-alkyl, and
G is
(i) O,
(ii) S,
(iii) NR²,
R¹ and R² are each independently or simultaneously
(i) H,
(ii) (C₁-C₆)-alkyl,
(iii) (C₃-C₁₀)-cycloalkyl,
(iv) (C₃-C₁₀)-heterocycloalkyl,
(v) -(CH₂)ₙ-(C₆-C₁₀)-aryl,
(vi) -(CH₂)ₙ-(C₅-C₁₀)-heteroaryl,
or
R¹ and R² are joined together to form a
(vii) guanine or a guanine derivative,
(viii) cytosine or a cytosine derivative,
(ix) thymine or a thymine derivative,
(x) adenine or an adenine derivative,
and Q is any suitable counteranion,
or a solvate, prodrug and/or stereoisomer thereof.

9. Compound for use according to claim 8, wherein the compound has the structure of formula (II) 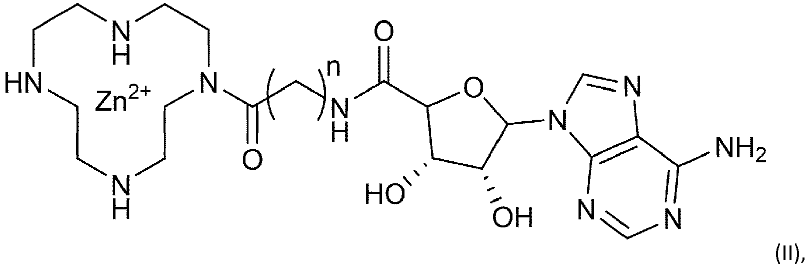 wherein n is an integer in the range of 1 to 5.

10. Compound for use according to claim 8, wherein the compound is DKM2-93 (CAS No. 65836-72-8, 2-chloro-N-(3,4-dimethoxybenzyl)acetamide).

11. Compound for use according to any one of claims 1 to 10, wherein ubiquitin-like modifier activating enzyme 5 (UBA5) comprises an amino acid sequence of any one of SEQ ID Nos:2-3, or an amino acid sequence having at least 80%, preferably at 90%, sequence identity to at least one of the sequences of SEQ ID NOs:2-3.

12. Pharmaceutical composition comprising the compound according to any one of claims 1 to 11, or its pharmaceutically acceptable salt, solvate, prodrug or ester, for use in a method of treating or preventing a disease or condition mediated by a flavivirus infection in a subject.

13. Pharmaceutical composition according to claim 12 which comprises one or more excipients, carriers and diluents.

14. *In vitro* method for identifying a pharmaceutically active compound for treating or preventing a disease or condition mediated by a flavivirus infection in a subject, comprising
(a) contacting a candidate compound with a ubiquitin-like modifier activating enzyme 5 (UBA5),
(b) detecting whether the candidate compound inhibits the function of UBA5,
wherein a compound which inhibits the function of UBA5, is suitable for treating or preventing a disease or condition mediated by a flavivirus infection in a subject.

15. *In vitro* method for identifying a pharmaceutically active compound for treating or preventing a disease or condition mediated by a flavivirus infection in a subject, comprising
(a) contacting a candidate compound with a cell that expresses a functional ubiquitin-like modifier activating enzyme 5 (UBA5),
(b) infecting the cell with a flavivirus before or after the cell was contacted with the candidate compound,
(c) detecting whether the candidate compound decreases the replication of the flavivirus in the cell, compared to a cell which was infected with the flavivirus but not contacted with the candidate compound,
wherein a compound which decreases the replication of a flavivirus in the cell, compared to a cell which was infected with the flavivirus but not contacted with the candidate compound, is suitable for treating or preventing a disease or condition mediated by a flavivirus infection in a subject.
